# EUROPÄISCHE PATENTSCHRIFT

(11) **EP 1 088 532 B1**
(45) Veröffentlichungstag und Bekanntmachung des Hinweises auf die Patenterteilung: **17.08.2005**
(21) Anmeldenummer: 00250327.4
(22) Anmeldetag: 28.09.2000
(51) Int. Cl.: A61F 2/40

(54) **Schultergelenk-Endoprothese**
Shoulder endoprosthesis
Endoprothèse d'épaule

(30) Priorität: 28.09.1999 DE 19948141
(43) Veröffentlichungstag der Anmeldung: 04.04.2001
(73) Patentinhaber: aap Implantate AG, 12099 Berlin (DE)
(72) Erfinder: Ahrens, Uwe, Dipl.-Ing., 12107 Berlin (DE)
(74) Vertreter: Meissner, Peter E., Dipl.-Ing.

(56) Entgegenhaltungen:
- EP-A- 0 053 633
- EP-A- 0 634 153
- WO-A-99/37254
- FR-A- 2 758 256
- US-A- 5 507 817
- US-A- 5 702 479
- US-A- 5 776 194

## Beschreibung

Die Erfindung betrifft eine Schultergelenk-Endoprothese, bestehend aus einem Kugelgelenkkopf und einem im Humerus verankerbaren Stielteil mit einem Prothesenkopf.

Eine Schultergelenk-Endoprothese dieser Art ist aus der DE 43 14 200 C1 bekannt. Hierbei geht es um die Schaffung einer Vollendoprothese mit einer künstlichen Rotatorenmanschette.

WO-A-9937254 beschreibt eine Humeruskopfprothese mit einem am Schaft angeordneten hohlen Köcher. Dieser Köcher ist mit zum Teil gewindetragenden Bohrungen versehen, die der exakten Positionierung der Tubercula dienen.

Bei einer Humeruskopftrümmerfraktur müssen die mit Tubercula abgelösten Sehnen der Rotatorenmanschette an der Prothese sicher befestigt werden. Diese Befestigung muß dauerhaft und belastbar sein, sowie die natürliche Bewegbarkeit wiederherstellen können.

Der Erfindung liegt daher die Aufgabe zugrunde, eine Schultergelenk-Endoprothese zu schaffen, mit der diese Art der Befestigung mit einfachen Mitteln erreichbar ist.

Gelöst wird diese Aufgabe erfindungsgemäß dadurch, dass der Prothesenkopf an seiner Außenoberfläche eine Vielzahl von Bohrungen aufweist, in die Tubercula-Bruchstücke arretierende Stifte kraft- und formschlüssig eintreibbar sind.

Vorzugsweise Ausgestaltungen ergeben sich aus den Unteransprüchen.

Die Öffnungen oder Bohrungen sind in großer Zahl und damit hoher Dichte in dem Bereich der Prothese angeordnet, der dem gesamten Bereich des Humeruskopfes entspricht. Durch die vorgesehene große Anzahl von derartigen Öffnungen oder Bohrungen lassen sich sie Tubercula-Bruchstücke sehr genau ausrichten bzw. positionieren, so dass die Bewegungsfähigkeit des Armes wieder voll erreichbar ist.

Die Tubercula-Bruchstücke werden zunächst an der entsprechenden Stelle des Prothesenkopfes auf diesen aufgelegt und dann wird ein Stift durch sie hindurch in die darunterliegende Bohrung eingetrieben. Dies geschieht mit einem definierten Impuls. Da der untere oder vordere Stiftbereich und die Bohrungen beispielsweise konisch ausgebildet sind, werden die Stifte kraft- und formschlüssig gehalten. Die überstehenden Enden der Stifte werden umgebogen. Durch diese erfindungsgemäße Lösung ist es möglich, die Rotatorenmanschette zu konservieren und ihre natürliche Lage zu rekonstruieren.

Da die Dichte der Öffnungen bzw. Bohrungen sehr groß ist, ist keinerlei Positionier - oder Zielhilfe notwendig, um eine passende Bohrung beim Eintreiben des Stiftes zu finden.

Die Stifte können vorzugsweise in Form dünner Drahtstifte ausgebildet sein, wobei zweckmäßig mehrere derartige Drahtstifte für die exakte Positionierung und Halterung jedes Tubercula-Bruchstücks sorgen. Hierdurch wird verhindert, dass das Tubercula-Bruchstück durch einen hohen Anpressdruck bzw. eine hohe Flächenpressung eines einzelnen Halteteiles nekrotisier wird.

Die Erfindung soll nachfolgend an den Zeichnungen erläutert werden. Dabei zeigt:
Figur 1 eine Seitenansicht des Stielteiles, teilweise geschnitten und
Figur 2 die Befestigung der Tubercula-Bruchstücke auf dem Prothesenkopf.

Die gesamte Endoprothese ist nicht dargestellt, da dies zur Erläuterung der Erfindung nicht notwendig ist.

Der Stielteil 1 der Prothese besteht aus dem Prothesenkopf 2 und den den eigentlichen Stiel bildenden Stielsegmenten 6. Die Stielsegmente sind ringförmig ausgebildet, wobei lediglich das Endteil eine von der Kreisform abweichende Form aufweist, um eine bessere Verankerung zu erreichen. Die einander zugewandten Ringflächen der Segmente weisen zahnartige Vorsprünge auf, die ineinandergreifen können, also komplementär sind.

Prothesenkopf und Stielsegmente werden durch eine koaxial zur Längsachse verlaufende Spannschraube 7 zusammengehalten, und zwar kraft- und formschlüssig. Auf diese Weise ist eine Verdrehsicherheit gegeben. Andererseits kann - nach Lösen der Spannschraube - eine rastermäßige Verdrehung der Teile relativ zueinander erfolgen, um eine optimale Ausrichtung des Prothesenkopfes zu erreichen. Die Anzahl der verwendeten Stielsegmente richtet sich nach der notwendigen Länge des Stiels.

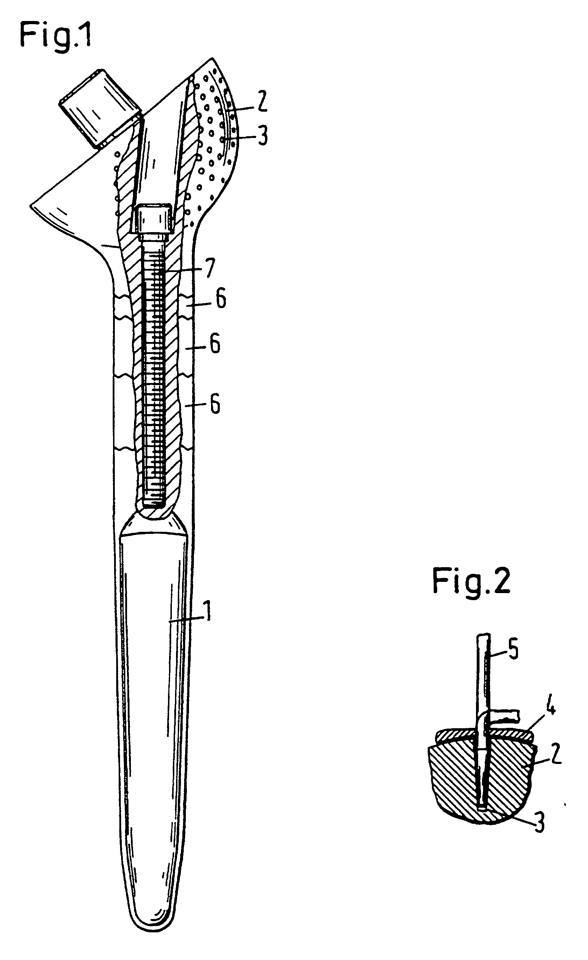

Die Figur 2 zeigt, wie die Tubercula-Bruchstücke 4 durch die Stifte 5 an der Außenoberfläche des Prothesenkopfes fixiert werden, wobei nach dem Eintreiben des Stiftes das überstehende Ende umgebogen wird.

## Patentansprüche

1. Schultergelenk - Endoprothese, bestehend aus einem Kugelgelenkkopf und einem im Humerus verankerbaren Stielteil (1) mit einem Prothesenkopf (2),
**dadurch gekennzeichnet,**
**dass** der Prothesenkopf (2) an seiner Außenoberfläche eine Vielzahl von Bohrungen (3) aufweist, so daß keinerlei Positionierungs - oder Zielhilfe notwendig ist, um eine passende Bohrung zu finden, in die Tubercula-Bruchstücke (4) arretierende Stifte (5) kraft- und formschlüssig eintreibbar sind.

2. Schultergelenk-Endoprothese nach Anspruch 1,
**dadurch gekennzeichnet,**
**dass** die Stifte (5) dünne Drahtstifte sind.

3. Schultergelenk - Endoprothese nach Anspruch 1,
**dadurch gekennzeichnet,**
**dass** die Stifte (5) und die Bohrungen (3) konische Endbereiche aufweisen.

4. Schultergelenk-Endoprothese nach einem der vorstehenden Ansprüche,
**dadurch gekennzeichnet,**
**dass** das Stielteil (1) modular aufgebaut ist, wobei der Prothesenkopf (2) mit dem aus einzelnen ringförmigen Stielsegmenten (6) bestehenden Stielteil (1) durch eine zur Stiellängsache koaxiale Spannschraube (7) miteinander verbunden sind.

5. Schultergelenk-Endoprothese nach Anspruch 4,
**dadurch gekennzeichnet,**
**dass** die einander zugewandten Ringflächen der Stielsegmente (6) zahnartig ausgebildet sind, so dass - nach Lösen der Spannschraube (7) - die Stielsegmente rastermäßig gegeneinander verdrehbar sind, während bei angezogener Spannschraube (7) eine kraft-und formschlüssige Verbindung besteht.

## Claims

1. Shoulder joint endoprosthesis, consisting of a ball-and-socket joint head and a shank part (1), which can be anchored in the humerus, with a prosthesis head (2),
**characterised in**
**that** the prosthesis head (2) comprises at its outer surface a plurality of bores (3), so that no positioning or aiming aid of any kind is necessary to find an appropriate bore, into which bores pins (5), which retain tubercula fragments (4), can be driven in a non-positive and positive manner.

2. Shoulder joint endoprosthesis according to Claim 1,
**characterised in**
**that** the pins (5) are thin wire pins.

3. Shoulder joint endoprosthesis according to Claim 1,
**characterised in**
**that** the pins (5) and the bores (3) comprise conical end regions.

4. Shoulder joint endoprosthesis according to any one of the preceding Claims,
**characterised in**
**that** the shank part (1) is of a modular structure, wherein the prosthesis head (2) is connected to the shank part (1), which consists of individual ring-shaped shank segments (6), by a clamping screw (7) which is coaxial with the longitudinal axis of the shank.

5. Shoulder joint endoprosthesis according to Claim 4,
**characterised in**
**that** the mutually facing ring faces of the shank segments (6) are of tooth-like formation, so that - after loosening the clamping screw (7) - the shank segments can be turned like a grid relative to one another, while a non-positive and positive connection is obtained when the clamping screw (7) is tightened.

## Revendications

1. Endoprothèse de l'articulation de l'épaule, comportant une tête à rotule et une partie tige (1) pouvant être ancrée dans l'humérus et ayant une tête de prothèse (2),
**caractérisée en ce que** la tête de prothèse (2) présente sur sa surface extérieure une pluralité de perçages (3), de telle manière qu'aucun outil de positionnement ou de visée n'est nécessaire pour trouver un perçage adéquat, dans lesquels perçages des broches (5) servant à fixer les fragments de tubercules (4) peuvent être introduites par adhérence et par correspondance de forme.

2. Endoprothèse de l'articulation de l'épaule selon la revendication 1,
**caractérisée en ce que** les broches (5) sont des broches métalliques fines.

3. Endoprothèse de l'articulation de l'épaule selon la revendication 1,
**caractérisée en ce que** les broches (5) et les perçages (3) présentent des zones d'extrémité coniques.

4. Endoprothèse de l'articulation de l'épaule selon l'une des revendications précédentes,
**caractérisée en ce que** la partie tige (1) est construite de façon modulaire, la tête de prothèse (2) étant reliée à la partie tige (1) composée de différents segments de tige (6) annulaires par une vis de serrage (7) coaxiale à l'axe longitudinal de tige.

5. Endoprothèse de l'articulation de l'épaule selon la revendication 4,
**caractérisée en ce que** les surfaces annulaires des segments de tige (6) orientées les unes vers les autres sont réalisées en forme de dents, de sorte que, après desserrage de la vis de serrage (7), les segments de tige peuvent être tournés par crans les uns par rapport aux autres, tandis que, lorsque la vis de serrage (7) est serrée, il se crée une liaison par adhérence et par correspondance de forme.
